# EUROPEAN PATENT APPLICATION

(11) **EP 4 776 265 A1**
(43) Date of publication of application: **15.07.2026**
(21) Application number: 24927682.5
(22) Date of filing: 15.11.2024
(51) Int. Cl.: G09B 23/28, G09B 9/00, G06F 3/01, G06T 19/00

(54) **ORAL TREATMENT UNIT TRAINING SYSTEM AND METHOD BASED ON SPATIAL COMPUTING TECHNIQUE**

(30) Priority: 05.11.2024 CN 202411573787
(71) Applicant: Beijing Unidraw VR Technology Research Institute Co., Ltd., Beijing 101399 (CN)
(72) Inventor: HAO, Aimin, Beijing 101399 (CN); ZHAO, Yongtao, Beijing 101399 (CN); HAN, Xianglong, Beijing 101399 (CN); ZHENG, Qinghua, Beijing 101399 (CN); FU, Haodong, Beijing 101399 (CN); ZHAO, Xiaohan, Beijing 101399 (CN); CONG, Yu, Beijing 101399 (CN); GENG, Shenghui, Beijing 101399 (CN); WANG, Shida, Beijing 101399 (CN); WAN, Jiawu, Beijing 101399 (CN)
(74) Representative: J A Kemp LLP
(86) International application number: PCT/CN2024/132502
(87) International publication number: WO 2026/097605

(57) **Abstract**

The present application provides a dental simulation workbench training system and method based on spatial computing technology, relating to augmented reality technology, for solving the problems that a simulator in the prior art only supports single-player use, only trains based on an oral model, lacks full-process doctor-patient communication, and has low simulation fidelity. By means of spatial computing technology, a virtual-real fusion head-mounted display acquires positioning data of a physical object in a real space and a dental chair of a diagnosis-treatment table. A situation observation server arranges a virtual entity and generates a scene file. A simulation computer generates a virtual-real fusion diagnosis-treatment scene, a virtual patient, and a diagnosis-treatment tool according to the scene file, which can simulate the whole process of a patient from entering the door to receiving diagnosis and treatment. Force sensation information is generated, and a pose of a force sensation tool in the force sensation information and a pose of a virtual diagnosis-treatment tool are controlled to match with a pose of a force feedback tool. A user wears the virtual-real fusion head-mounted display to perform a diagnosis-treatment operation. By means of network synchronization technology, multiple people are supported to watch the operation process of the same diagnosis-treatment table. The present application improves the comprehensive simulation fidelity.

## Description

The present application claims priority to Chinese Patent Application No. 202411573787.0, filed with the China National Intellectual Property Administration on November 05, 2024 and entitled "DENTAL SIMULATION WORKBENCH TRAINING SYSTEM AND METHOD BASED ON SPATIAL COMPUTING TECHNOLOGY", which is incorporated herein by reference in its entirety.

### TECHNICAL FIELD

The present application relates to the field of augmented reality technology, and in particular, to a dental simulation workbench training system and method based on spatial computing technology.

### BACKGROUND

With the rapid development of augmented reality technology, it has been widely used in multiple fields. Establishing a dental simulation workbench training system through augmented reality technology can effectively help doctors carry out oral diagnosis-treatment training.

In the prior art, there is an oral surgical skill training simulator, which includes a simulation training platform, an oral operation training system based on a force feedback device, and an observation system based on an augmented reality headset. Here, the simulation training platform is used to generate a simulated head model, the oral operation training system based on the force feedback device is used to generate a specific force to simulate the force sensation when touching an oral cavity, and the observation system based on the augmented reality headset is used to generate a corresponding virtual training visual according to the simulated head model and the force sensation.

However, there is a gap between the prior art and a real clinical operation, with low simulation fidelity, which affects the user experience.

### SUMMARY

Embodiments of the present application provide a dental simulation workbench training system and method based on spatial computing technology, aiming to achieve the technical effect of improving the simulation fidelity.

In a first aspect, the present application provides a dental simulation workbench training system based on spatial computing technology, including: a plurality of diagnosis-treatment tables and a situation observation server; where the plurality of diagnosis-treatment tables communicate with the situation observation server through a TCP/IP protocol, and each of the diagnosis-treatment tables includes: a simulation computer, a virtual-real fusion head-mounted display, and a dual-hand force feedback tool;
the virtual-real fusion head-mounted display is configured to acquire positioning data of a physical object existing in a real space and a dental chair of a diagnosis-treatment table based on spatial computing technology, where the physical object does not include the dental chair of the diagnosis-treatment table;
the situation observation server is configured to generate a scene file for performing oral diagnosis-treatment according to the positioning data;
the simulation computer is configured to: load the scene file, generate a virtual-real fusion space according to the scene file, load a pre-created virtual patient to be diagnosed-and-treated and a virtual diagnosis-treatment tool corresponding to a real diagnosis-treatment tool in the virtual-real fusion space, and record simulated entity data of the virtual patient and the virtual diagnosis-treatment tool;
the simulation computer is further configured to: generate a virtual diagnosis-treatment scene according to the scene file and the simulated entity data; generate force sensation information according to a diagnosis-treatment operation performed by a real user through operating the dual-hand force feedback tool, where the force sensation information includes pose information of a force sensation tool; and control a pose of the force sensation tool and a pose of the virtual diagnosis-treatment tool to match with a pose of the dual-hand force feedback tool based on a matching algorithm, to obtain matched simulated entity data;
the virtual-real fusion head-mounted display is configured to generate a first virtual-real fusion diagnosis-treatment visual according to the matched simulated entity data, where the first virtual-real fusion diagnosis-treatment visual is used for the real user to perform the diagnosis-treatment operation;
the situation observation server is further configured to acquire simulated entity data corresponding to any one of the diagnosis-treatment tables;
the virtual-real fusion head-mounted display is further configured to generate a second virtual-real fusion diagnosis-treatment visual according to the simulated entity data of the any one of the diagnosis-treatment tables forwarded by the situation observation server;
where in both the first virtual-real fusion diagnosis-treatment visual and the second virtual-real fusion diagnosis-treatment visual, the virtual patient undergoing the diagnosis-treatment operation is shown to be closely aligned with the dental chair of the diagnosis-treatment table in the real space.

Optionally, when configured to acquire the positioning data of the physical object existing in the real space and the dental chair of the diagnosis-treatment table based on spatial computing technology, the virtual-real fusion head-mounted display is specifically configured to:
scan a positioning picture with a significant feature at a specified position in the real space to determine a reference coordinate system and a reference point;
acquire pose information of the reference point in a current device coordinate system;
establish a mapping relationship between the reference coordinate system and the device coordinate system according to the pose information of the reference point in the current device coordinate system;
measure positioning data of the physical object existing in the real space in the reference coordinate system;
scan a positioning picture with a significant feature at a position of a positioning benchmark point of the dental chair of the diagnosis-treatment table in the real space, to acquire pose information of the dental chair of the diagnosis-treatment table in the device coordinate system;
obtain positioning data of the dental chair of the diagnosis-treatment table in the reference coordinate system according to the pose information of the dental chair of the diagnosis-treatment table in the device coordinate system and the mapping relationship.

Optionally, when configured to generate the scene file for performing oral diagnosis-treatment according to the positioning data, the situation observation server is specifically configured to:
generate a virtual space according to the positioning data of the physical object in the reference coordinate system;
generate a virtual dental chair of a diagnosis-treatment table at a corresponding position in the virtual space according to the positioning data of the dental chair of the diagnosis-treatment table in the reference coordinate system;
generate a preset virtual facility in the virtual space in response to a user's operation;
acquire coordinate-pose data of the virtual space, the virtual dental chair of the diagnosis-treatment table, and the virtual facility, and generate the scene file for performing oral diagnosis-treatment according to the coordinate-pose data.

Optionally, when configured to generate the force sensation information according to the diagnosis-treatment operation performed by the real user through operating the dual-hand force feedback tool, where the force sensation information includes the pose information of the force sensation tool, and control the pose of the force sensation tool and the pose of the virtual diagnosis-treatment tool to match with the pose of the dual-hand force feedback tool based on the matching algorithm, to obtain the matched simulated entity data, the simulation computer is specifically configured to:
determine a benchmark point of a force sensation coordinate system and the force sensation coordinate system according to a preset position of the dental chair of the diagnosis-treatment table;
acquire pose information of a head model of the virtual patient in the force sensation coordinate system;
generate the force sensation information according to the performed diagnosis-treatment operation of the dual-hand force feedback tool based on the pose information of the head model in the force sensation coordinate system, where the force sensation information includes the pose information of the force sensation tool;
acquire pose information of the virtual diagnosis-treatment tool in the force sensation coordinate system;
in the force sensation coordinate system, match the pose information of the force sensation tool and the pose information of the virtual diagnosis-treatment tool in the force sensation coordinate system with pose information of the dual-hand force feedback tool, and convert the matched pose information to the reference coordinate system, to obtain matched simulated entity data in the reference coordinate system.

Optionally, when configured to generate the first virtual-real fusion diagnosis-treatment visual according to the matched simulated entity data, the virtual-real fusion head-mounted display is specifically configured to:
acquire the mapping relationship between the reference coordinate system and the device coordinate system;
convert the matched simulated entity data in the reference coordinate system to the device coordinate system according to the mapping relationship, to obtain simulated entity data in the device coordinate system;
acquire the scene file, and obtain a virtual visual in the virtual-real fusion space according to the scene file and the simulated entity data in the device coordinate system;
acquire a real-time visual captured in the real space;
perform superposition processing on the virtual visual in the virtual-real fusion space and the real-time visual, to obtain the first virtual-real fusion diagnosis-treatment visual.

Optionally, the situation observation server is further configured to:
display virtual diagnosis-treatment scenes corresponding to different diagnosis-treatment tables.

Optionally, the situation observation server is further configured to:
set a virtual patient number-calling mode, where the number-calling mode includes a normal number-calling mode and an enhanced number-calling mode, where a virtual patient corresponding to the normal number-calling mode has a random disease cause, and a virtual patient corresponding to the enhanced number-calling mode has a preset fixed disease cause;
edit a virtual patient queue corresponding to the number-calling mode.

Optionally, the diagnosis-treatment table further includes: a speaker and a microphone;
after the simulation computer loads the pre-created virtual patient to be diagnosed-and-treated in the virtual-real fusion space, the real user sends a control instruction to the virtual patient through the microphone, and receives an interactive voice output by the virtual patient through the speaker.

Optionally, the diagnosis-treatment table further includes: a height sensor and a pitch sensor;
when the simulation computer detects that a lifting height of the dental chair measured by the height sensor or a rotation angle of the dental chair measured by the pitch sensor changes, the simulation computer controls, based on the matching algorithm, the pose of the force sensation tool and the pose of the virtual diagnosis-treatment tool to match with the pose of the dual-hand force feedback tool again, to obtain new matched simulated entity data.

Optionally, the dental simulation workbench training system based on spatial computing technology is connected to at least one external handheld terminal, so that the at least one external handheld terminal displays a virtual-real fusion diagnosis-treatment visual, where the at least one external terminal has an augmented reality function.

In a second aspect, the present application provides an oral diagnosis-treatment training method based on spatial computing technology, applied to a dental simulation workbench training system based on spatial computing technology, where the method includes:
acquiring positioning data of a physical object existing in a real space and a dental chair of a diagnosis-treatment table based on spatial computing technology, where the physical object does not include the dental chair of the diagnosis-treatment table;
generating a scene file for performing oral diagnosis-treatment according to the positioning data;
loading the scene file, generating a virtual-real fusion space according to the scene file, loading a pre-created virtual patient to be diagnosed-and-treated and a virtual diagnosis-treatment tool corresponding to a real diagnosis-treatment tool in the virtual-real fusion space, and recording simulated entity data of the virtual patient and the virtual diagnosis-treatment tool;
generating a virtual diagnosis-treatment scene according to the scene file and the simulated entity data; generating force sensation information according to a diagnosis-treatment operation performed by a real user through operating a dual-hand force feedback tool, where the force sensation information includes pose information of a force sensation tool; and controlling a pose of the force sensation tool and a pose of the virtual diagnosis-treatment tool to match with a pose of the dual-hand force feedback tool based on a matching algorithm, to obtain matched simulated entity data;
generating a first virtual-real fusion diagnosis-treatment visual according to the matched simulated entity data, where the first virtual-real fusion diagnosis-treatment visual is used for the real user to perform the diagnosis-treatment operation;
acquiring simulated entity data corresponding to any diagnosis-treatment table;
generating a second virtual-real fusion diagnosis-treatment visual according to the forwarded simulated entity data of the any diagnosis-treatment table;
where in both the first virtual-real fusion diagnosis-treatment visual and the second virtual-real fusion diagnosis-treatment visual, the virtual patient undergoing the diagnosis-treatment operation is shown to be closely aligned with the dental chair of the diagnosis-treatment table in the real space.

Optionally, the acquiring the positioning data of the physical object existing in the real space and the dental chair of the diagnosis-treatment table based on spatial computing technology includes:
scanning a positioning picture with a significant feature at a specified position in the real space to determine a reference coordinate system and a reference point;
acquiring pose information of the reference point in a current device coordinate system;
establishing a mapping relationship between the reference coordinate system and the device coordinate system according to the pose information of the reference point in the current device coordinate system;
measuring positioning data of the physical object existing in the real space in the reference coordinate system;
scanning a positioning picture with a significant feature at a position of a positioning benchmark point of the dental chair of the diagnosis-treatment table in the real space, to acquire pose information of the dental chair of the diagnosis-treatment table in the device coordinate system;
obtaining positioning data of the dental chair of the diagnosis-treatment table in the reference coordinate system according to the pose information of the dental chair of the diagnosis-treatment table in the device coordinate system and the mapping relationship.

Optionally, the generating the scene file for performing oral diagnosis-treatment according to the positioning data includes:
generating a virtual space according to the positioning data of the physical object in the reference coordinate system;
generating a virtual dental chair of the diagnosis-treatment table at a corresponding position in the virtual space according to the positioning data of the dental chair of the diagnosis-treatment table in the reference coordinate system;
generating a preset virtual facility in the virtual space in response to a user's operation;
acquiring coordinate-pose data of the virtual space, the virtual dental chair of the diagnosis-treatment table, and the virtual facility, and generating the scene file for performing oral diagnosis-treatment according to the coordinate-pose data.

Optionally, the generating the force sensation information according to the diagnosis-treatment operation performed by the real user through operating the dual-hand force feedback tool, where the force sensation information includes the pose information of the force sensation tool, and controlling the pose of the force sensation tool and the pose of the virtual diagnosis-treatment tool to match with the pose of the dual-hand force feedback tool based on the matching algorithm, to obtain the matched simulated entity data, includes:
determining a benchmark point of a force sensation coordinate system and the force sensation coordinate system according to a preset position of the dental chair of the diagnosis-treatment table;
acquiring pose information of a head model of the virtual patient in the force sensation coordinate system;
generating the force sensation information according to the performed diagnosis-treatment operation of the dual-hand force feedback tool based on the pose information of the head model in the force sensation coordinate system, where the force sensation information includes the pose information of the force sensation tool;
acquiring the pose information of the virtual diagnosis-treatment tool in the force sensation coordinate system;
in the force sensation coordinate system, matching the pose information of the force sensation tool and the pose information of the virtual diagnosis-treatment tool in the force sensation coordinate system with pose information of the dual-hand force feedback tool, and converting the matched pose information to the reference coordinate system, to obtain matched simulated entity data in the reference coordinate system.

Optionally, the generating the first virtual-real fusion diagnosis-treatment visual according to the matched simulated entity data includes:
acquiring the mapping relationship between the reference coordinate system and the device coordinate system;
converting the matched simulated entity data in the reference coordinate system to the device coordinate system according to the mapping relationship, to obtain simulated entity data in the device coordinate system;
acquiring the scene file, and obtaining a virtual visual in the virtual-real fusion space according to the scene file and the simulated entity data in the device coordinate system;
acquiring a real-time visual captured in the real space;
performing superposition processing on the virtual visual in the virtual-real fusion space and the real-time visual, to obtain the first virtual-real fusion diagnosis-treatment visual.

The embodiments of the present application provide a dental simulation workbench training system and method based on spatial computing technology. The system includes: the plurality of diagnosis-treatment tables and a situation observation server, where the plurality of diagnosis-treatment tables communicate with the situation observation server through the TCP/IP protocol, and each of the diagnosis-treatment tables includes: the simulation computer, the virtual-real fusion head-mounted display, and the dual-hand force feedback tool. The virtual-real fusion head-mounted display acquires the positioning data of the physical object existing in the real space and the dental chair of the diagnosis-treatment table based on spatial computing technology. The situation observation server generates the scene file for performing oral diagnosis-treatment according to the positioning data. The simulation computer loads the scene file, generates the virtual-real fusion space according to the scene file, loads the pre-created virtual patient to be diagnosed-and-treated and the virtual diagnosis-treatment tool corresponding to the real diagnosis-treatment tool in the virtual-real fusion space, and records the simulated entity data of the virtual patient and the virtual diagnosis-treatment tool. The simulation computer further generates the virtual diagnosis-treatment scene according to the scene file and the simulated entity data, and acquires the force sensation information according to the diagnosis-treatment operation performed by the real user through operating the dual-hand force feedback tool, where the force sensation information includes the pose information of the force sensation tool, and then controls the pose of the force sensation tool and the pose of the virtual diagnosis-treatment tool to match with the pose of the dual-hand force feedback tool based on the matching algorithm, to obtain the matched simulated entity data. The virtual-real fusion head-mounted display generates the first virtual-real fusion diagnosis-treatment visual according to the matched simulated entity data. The situation observation server may further acquire the simulated entity data corresponding to any one of the diagnosis-treatment tables, so that the virtual-real fusion head-mounted display may generate the corresponding second virtual-real fusion diagnosis-treatment visual according to the simulated entity data of the any one of the diagnosis-treatment tables forwarded by the situation observation server. In the present application, by generating the virtual-real fusion diagnosis-treatment scene, the simulation fidelity is improved, and the system can support multiple people to watch the diagnosis-treatment process of the same diagnosis-treatment table, which is convenient for users to communicate and improves the user experience.

### BRIEF DESCRIPTION OF DRAWINGS

The accompanying drawings herein are incorporated into and form a part of this description, illustrate embodiments consistent with the present application, and are used together with the description to explain the principles of the present application.
FIG. 1 is a schematic structural diagram of a dental simulation workbench training system based on spatial computing technology provided in an embodiment of the present application.
FIG. 2 is a schematic flow chart of a method for acquiring positioning data of a physical object existing in a real space and a dental chair of a diagnosis-treatment table based on spatial computing technology provided in an embodiment of the present application.
FIG. 3 is a schematic flow chart of a method for obtaining matched simulated entity data provided in an embodiment of the present application.
FIG. 4 is a schematic diagram after virtual-real matching provided in an embodiment of the present application.
FIG. 5 is a schematic diagram of a dental simulation workbench training system based on spatial computing technology provided in an embodiment of the present application.
FIG. 6 is a schematic application diagram of a dental simulation workbench training system based on spatial computing technology provided in an embodiment of the present application.
FIG. 7 is a schematic scene diagram provided in an embodiment of the present application.
FIG. 8 is a schematic flow chart of an oral diagnosis-treatment training method based on spatial computing technology provided in an embodiment of the present application.

Through the above accompanying drawings, the specific embodiments of the present application have been shown, and more detailed descriptions will be provided hereinafter. These accompanying drawings and text descriptions are not intended to limit the scope of the concept of the present application in any way, but to explain the concept of the present application to those skilled in the art by referring to specific embodiments.

### DESCRIPTION OF EMBODIMENTS

Here, the exemplary embodiments will be described in detail, and examples thereof are shown in the drawings. When the following description relates to the drawings, unless otherwise indicated, the same numbers in different drawings represent the same or similar elements. The implementations described in the following exemplary embodiments do not represent all implementations consistent with the present application. On the contrary, they are only examples of apparatuses and methods consistent with some aspects of the present application as detailed in the appended claims.

In the description of the embodiments of the present application, terms indicating directions or positional relationships such as "inside" and "outside" are based on the directions or positional relationships shown in the drawings. They are merely for convenience of description, and do not indicate or imply that the apparatus or component must have a specific orientation, be constructed and operated in a specific orientation. Therefore, it should not be construed as a limitation to the present application.

In the description of the embodiments of the present application, unless otherwise specifically specified and limited, the terms "connected" and "connection" should be understood in a broad sense. For example, it may be a fixed connection, a detachable connection, or an integral connection; it may be a mechanical connection or an electrical connection; it may be directly connected, or indirectly connected through an intermediate medium, and it may be an internal communication of two components. For those skilled in the art, the specific meaning of the above terms in the embodiments of the present application can be understood according to specific situations.

It should be noted that the user information (including but not limited to user device information, user personal information, etc.) and data (including but not limited to data for analysis, stored data, displayed data, etc.) involved in the present application are all information and data authorized by the user or fully authorized by all parties. Moreover, the collection, use, and processing of relevant data need to comply with the relevant laws, regulations, and standards of the relevant countries and regions, and corresponding operation entrances shall be provided for users to select authorization or refusal.

Mastery of oral clinical skills requires medical students or doctors to undergo a large number of practical operation trainings. However, in traditional teaching, medical students have limited opportunities for hands-on practice. Junior doctors also often face the dilemma of lacking patients for practical exercises in the early stages of their careers. This poses a challenge to their skill development and confidence building.

In the prior art, in order to improve the diagnosis-treatment skills of medical students or doctors, training is usually carried out by using a surgical skill training simulator to simulate the diagnosis-treatment scene. The simulator of this type usually includes a support platform, a simulation computer, a display screen, a force feedback device, etc. Here, the support platform has a specific structure, integrating various devices to form an integrated simulator. The simulation computer is built into the support platform, and is used to provide simulation control of the diagnosis-treatment surgical operation process, as well as output visual and force sensation information. The output virtual 3D oral model is displayed on the display screen for the user to view. The output force sensation information is transmitted through the force feedback device and a series of connecting rods, and finally perceived by the hand-held part (dental instrument) at the end of the connecting rod in the user's hand. The hand-held part at the end of the connecting rod is usually located below the display screen and is in the same space as the virtual 3D oral model presented on the display screen in perception, so as to simulate the real diagnosis-treatment process.

Exemplarily, there is currently an oral surgical skill training simulator, which includes a simulation training platform, an oral operation training system based on a force feedback device, and an observation system based on an augmented reality headset. The simulation training platform is used to generate a simulated head model. The oral operation training system based on the force feedback device is used to generate a specific force to simulate the force sensation when touching the oral cavity. The observation system based on the augmented reality headset is used to generate a corresponding virtual training visual according to the simulated head model and the force sensation.

However, the current training simulator only supports single-player use. It cannot enable multiple people to watch the diagnosis-treatment operation of the same diagnosis-treatment table at the same time, which is not convenient for users to communicate in a timely manner. Moreover, the training is only based on the oral model, without considering the patient's reception and communication process. There is a lack of practice in communication skills with the patient and in diagnosis. In addition, the entire virtual space of a diagnosis-treatment room has not been constructed, resulting in a relatively low simulation fidelity. There is a lack of on-site immersion, which affects the user experience.

Therefore, in view of the above technical problems in the prior art, during the research process, the inventor found that based on spatial computing technology, by superimposing a virtual patient in a real space and fusing the real space with a virtual space, the obtained virtual-real fusion diagnosis-treatment visual can be made closer to reality, thereby improving the simulation fidelity. By enabling multiple people to watch the virtual-real fusion diagnosis-treatment visual of the same diagnosis-treatment table at the same time, it is convenient for users to communicate in a timely manner, thus enhancing the user experience. Based on this, the present application provides a dental simulation workbench training system and method based on spatial computing technology.

The application scenario of the dental simulation workbench training system based on spatial computing technology provided in the present application can be applied in schools for teaching, in hospitals for training, or in other scenarios with needs, which is not limited in the present application.

The following uses specific embodiments to describe in detail the technical solutions of the present application and how the technical solutions of the present application solve the above technical problems. The following specific embodiments may be combined with each other, and the same or similar concepts or processes may not be repeated in some embodiments. The following will describe the embodiments of the present application in combination with the drawings.

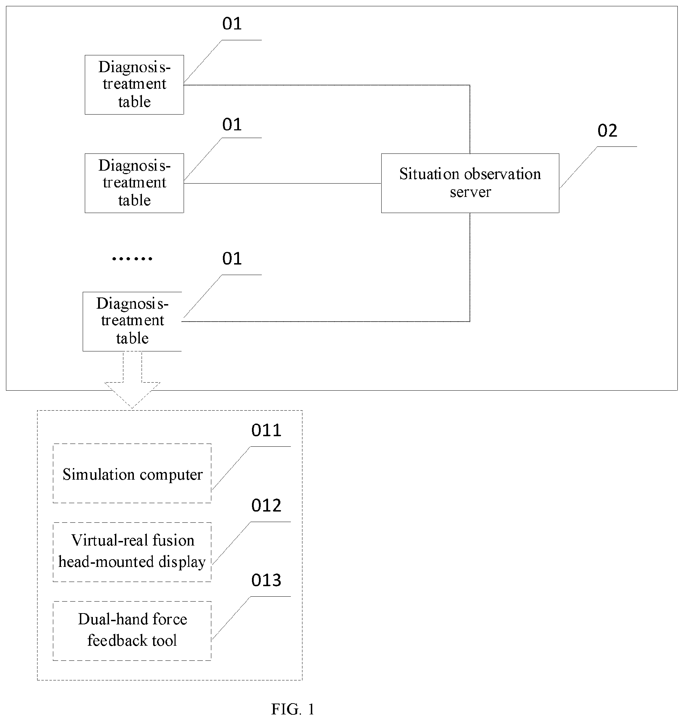

FIG. 1 is a schematic structural diagram of a dental simulation workbench training system based on spatial computing technology provided in an embodiment of the present application. As shown in FIG. 1, the system includes: a plurality of diagnosis-treatment tables 01 and a situation observation server 02, and each of the diagnosis-treatment tables 01 communicates with the situation observation server 02 through a TCP/IP protocol.

Here, each of the diagnosis-treatment tables includes: a simulation computer 011, a virtual-real fusion head-mounted display 012, and a dual-hand force feedback tool 013.

The virtual-real fusion head-mounted display 012 is configured to acquire positioning data of a physical object existing in a real space and a dental chair of a diagnosis-treatment table 01 based on spatial computing technology, where the physical object does not include the dental chair of the diagnosis-treatment table 01.

In this embodiment, the virtual-real fusion head-mounted display 012 may be a mixed reality device, such as a mixed display headset, augmented reality glasses, etc., with spatial computing function, capable of object positioning, generating a virtual visual and superimposing it with a real visual for display, and having wireless transmission function.

A scene-setting tool based on spatial computing technology is installed in the virtual-real fusion head-mounted display 012. The scene-setting tool is an application component of a spatial computing technology scene-setting system, and is used to measure a position of a physical object existing in the real space, i.e., a real diagnosis-treatment room, and is further used to locate the pose of the dental chair of the diagnosis-treatment table 01 in a created virtual space.

Optionally, the physical object may be each wall, floor, ceiling, door, etc. It can be understood that the type of the physical object is not limited in this embodiment, and may further be other things existing in the real space.

After acquiring the positioning data of the physical object existing in the real space and the dental chair of the diagnosis-treatment table 01, the virtual-real fusion head-mounted display 012 communicates the positioning data to the situation observation server 02 through the TCP/IP protocol.

The situation observation server 02 is configured to generate a scene file for performing oral diagnosis-treatment according to the positioning data.

During a scene arrangement stage, the situation observation server 02 may receive the positioning data of the physical object and the dental chair of the diagnosis-treatment table 01 sent by the virtual-real fusion head-mounted display 012, generate the virtual space according to the positioning data, and arrange a virtual facility related to the diagnosis-treatment room in the virtual space, such as a medical cabinet, a number-calling screen, etc.

The situation observation server 02 may further generate the scene file according to generated coordinate-pose data of the virtual space, the virtual dental chair of the diagnosis-treatment table, and the virtual facility, and save the scene file in a preset storage module.

In this embodiment, the situation observation server 02 may further set a virtual patient number-calling mode and edit a virtual patient queue corresponding to the number-calling mode. The number-calling mode may include a normal number-calling mode and an enhanced number-calling mode. Here, the virtual patient corresponding to the normal number-calling mode has a random disease cause, and the virtual patient corresponding to the enhanced number-calling mode has a preset fixed disease cause.

In the normal number-calling mode, all of the diagnosis-treatment tables 01 share the same virtual patient queue with random disease causes. When a diagnosis-treatment table 01 calls a number, a patient can be dequeued from the shared queue in sequence for diagnosis. In the enhanced number-calling mode, each of the diagnosis-treatment tables 01 corresponds to a dedicated queue of a specified disease cause sequence for enhanced practice.

The situation observation server 02 is further equipped with a large display screen. When receiving number-calling information sent by a diagnosis-treatment table 01, the situation observation server 02 may determine a target virtual patient from the virtual patient queue corresponding to the number-calling mode according to the number-calling mode set by the situation observation server 02 and the identification information of the diagnosis-treatment table 01 included in the number-calling information, and sent to the corresponding diagnosis-treatment table 01. The large display screen will also display the target virtual patient.

The simulation computer 011 is configured to: load the scene file, generate a virtual-real fusion space according to the scene file, load a pre-created virtual patient to be diagnosed-and-treated and a virtual diagnosis-treatment tool corresponding to a real diagnosis-treatment tool in the virtual-real fusion space, and record simulated entity data of the virtual patient and the virtual diagnosis-treatment tool.

In this embodiment, the simulation computer 011 may access the situation observation server 02 in a wired manner for communication connection, so that it can load the scene file distributed by the situation observation server 02.

An oral diagnosis-treatment simulation training system runs on the simulation computer 011.

The simulation computer 011 loads the scene file distributed by the situation observation server 02, and generates the virtual-real fusion space according to the scene file to simulate the diagnosis-treatment room for performing diagnosis-treatment training.

The simulation computer 011 loads the pre-created virtual patient to be diagnosed-and-treated through the number-calling operation, and loads a virtual oral model corresponding to the virtual patient. Here, the virtual oral model has been pre-constructed based on oral data. For example, the oral data may be cone beam CT (Cone Beam CT, CBCT) scan data and true color scan data, etc.

Optionally, the oral data of the virtual patient may be acquired in advance by collecting the oral data of a real patient. For example, by using a CBCT device and a true color scanning device to scan the patient's oral cavity, the CBCT oral data in DICOM format and the oral scan data in stl format are obtained. Through a preset three-dimensional modeling model, superimposed reconstruction is performed based on the above data to obtain a complete three-dimensional surface and physical model of a lower half of the skull, thereby the virtual oral model is pre-constructed.

Optionally, a virtual oral operation environment may be pre-created according to the virtual oral model and the virtual diagnosis-treatment tool. The virtual diagnosis-treatment tool may be pre-created according to the acquired measurement data of the diagnosis-treatment tool, such as the size, shape, etc.

Optionally, the virtual patient may be pre-created. Specifically, a diagnosis-treatment information set for the virtual patient is constructed from a pre-constructed instruction-based behavior-driven model and a knowledge graph model. The diagnosis-treatment information set is fused with the virtual oral operation environment. Finally, the virtual patient is generated, where the virtual patient has the capability of force feedback interaction.

The simulation computer 011 records the simulated entity data of the virtual patient and the virtual diagnosis-treatment tool, and shares the simulated entity data to the situation observation server 02.

In this embodiment, each simulation computer 011 will be assigned a unique Id_{Sim}. When the simulation computer 011 loads the virtual patient and the virtual diagnosis-treatment tool, a local unique Id_{Local} will be assigned to the loaded virtual patient and the virtual diagnosis-treatment tool. A global unique Id_{Global} is generated for the loaded virtual patient and the virtual diagnosis-treatment tool based on a preset method of Id_{Sim} << 16 | Id_{Local}, as a synchronization identifier for the unified virtual-real fusion space.

The simulation computer 011 is configured to: generate a virtual diagnosis-treatment scene according to the scene file and the simulated entity data, and acquire force sensation information according to a diagnosis-treatment operation performed by a real user through operating the dual-hand force feedback tool 013. The force sensation information includes pose information of a force sensation tool, and further includes the magnitude and direction of the force, etc.

The simulation computer 011 generates the virtual diagnosis-treatment scene according to the loaded scene file and the created simulated entity, where each simulated entity has a unique Id_{Global} identifier.

The simulation computer 011 is further configured to control a pose of the force sensation tool and a pose of the virtual diagnosis-treatment tool to match with a pose of the dual-hand force feedback tool based on a matching algorithm, to obtain matched simulated entity data.

After the simulation computer 011 loads the virtual patient and the virtual diagnosis-treatment tool, it acquires the force sensation information by performing diagnosis-treatment operation on the virtual patient via the dual-hand force feedback tool 013. Furthermore, based on a preset triple matching algorithm, a pose of the force sensation tool and a pose of the virtual diagnosis-treatment tool are controlled to match with a pose of the dual-hand force feedback tool, so that a presented virtual-real diagnosis-treatment visual is closer to reality.

Subsequently, the virtual-real fusion head-mounted display 012 generates a first virtual-real fusion diagnosis-treatment visual according to the matched simulated entity data.

The virtual-real fusion head-mounted display 012 first detects whether an object corresponding to the identifier Id_{Global} exists. If it is true, the existing object is updated according to the acquired simulated entity data. If it is false, a corresponding object is created according to the acquired simulated entity data. Finally, a virtual-real fusion diagnosis-treatment visual is obtained.

In the present application, the situation observation server 02 may further acquire the simulated entity data of a simulation computer from any one of the diagnosis-treatment tables, so that the virtual-real fusion head-mounted display 012 generates a corresponding virtual-real fusion diagnosis-treatment visual according to the simulated entity data of the any one of the diagnosis-treatment tables forwarded by the situation observation server 02.

In the generated virtual-real fusion diagnosis-treatment visual, the virtual patient undergoing the diagnosis-treatment operation will be shown to be closely aligned with the dental chair of the diagnosis-treatment table in the real space.

By communicating based on the TCP/IP protocol, that is, multi-player network synchronization technology, the plurality of diagnosis-treatment tables are networked for use. This enables any real user wearing the virtual-real fusion head-mounted display 012 to observe virtual-real fusion diagnosis-treatment visuals of other diagnosis-treatment tables at any time, facilitating timely communication among users and thus enhancing the user experience.

In the present application, the large display screen equipped on the situation observation server 02 may further display the virtual diagnosis-treatment scene corresponding to any one of the diagnosis-treatment tables.

In the above embodiment of the present application, the system includes: a plurality of diagnosis-treatment tables and a situation observation server, where the plurality of diagnosis-treatment tables communicate with the situation observation server through the TCP/IP protocol, and each of the diagnosis-treatment tables includes: a simulation computer, a virtual-real fusion head-mounted display, and a dual-hand force feedback tool. The virtual-real fusion head-mounted display acquires the positioning data of the physical object existing in the real space and the dental chair of the diagnosis-treatment table based on spatial computing technology. The situation observation server generates the scene file for performing oral diagnosis-treatment according to the positioning data. The simulation computer loads the scene file, generates the virtual-real fusion space according to the scene file, loads the pre-created virtual patient to be diagnosed-and-treated and the virtual diagnosis-treatment tool corresponding to the real diagnosis-treatment tool in the virtual-real fusion space, and records the simulated entity data of the virtual patient and the virtual diagnosis-treatment tool. The simulation computer further generates the virtual diagnosis-treatment scene according to the scene file and the simulated entity data, and acquires the force sensation information according to the diagnosis-treatment operation performed by the real user through operating the dual-hand force feedback tool, where the force sensation information includes the pose information of the force sensation tool, and then controls the pose of the force sensation tool and the pose of the virtual diagnosis-treatment tool to match with the pose of the dual-hand force feedback tool based on the matching algorithm, to obtain the matched simulated entity data. The virtual-real fusion head-mounted display generates the first virtual-real fusion diagnosis-treatment visual according to the matched simulated entity data. The situation observation server may further acquire the simulated entity data corresponding to any one of the diagnosis-treatment tables, so that the virtual-real fusion head-mounted display may generate the corresponding second virtual-real fusion diagnosis-treatment visual according to the simulated entity data of the any one of the diagnosis-treatment tables forwarded by the situation observation server. In the system of the embodiment, by generating the virtual-real fusion diagnosis-treatment scene, the simulation fidelity is improved, and the system can support multiple people to watch the diagnosis-treatment process of the same diagnosis-treatment table, which is convenient for users to communicate and improves the user experience.

Furthermore, on the basis of the above embodiment, the following embodiment illustrates the process in which the virtual-real fusion head-mounted display is used to acquire the positioning data of the physical object existing in the real space and the dental chair of the diagnosis-treatment table based on spatial computing technology.

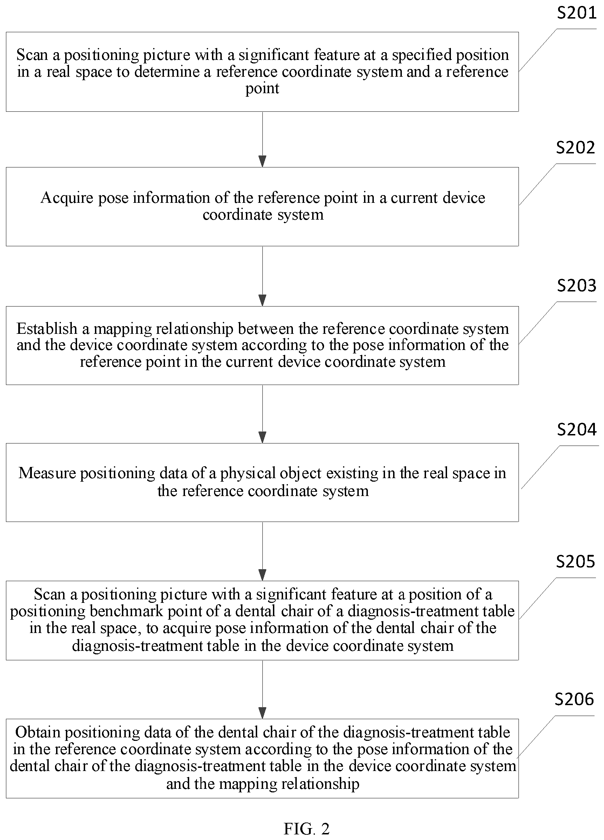

FIG. 2 is a schematic flow chart of a method for acquiring positioning data of a physical object existing in a real space and a dental chair of a diagnosis-treatment table based on spatial computing technology provided in an embodiment of the present application. As shown in FIG. 2, the method may include the following.

S201, scan a positioning picture with a significant feature at a specified position in the real space to determine a reference coordinate system and a reference point.

S202, acquire pose information of the reference point in a current device coordinate system.

A position is selected in advance within a real empty room Rᵣₑₐₗ as the reference point O_{ref}, and the orientation of each coordinate axis is specified to form the reference coordinate system Coord_{ref}. A Marker picture is placed at the reference point O_{ref}. Here, the Marker picture may be a QR (Quick Response) code image or other pictures with obvious features.

When the virtual-real fusion head-mounted display is activated, the device coordinate system Coord_{dev} and the origin O_{dev} of the current device are determined.

The virtual-real fusion head-mounted display scans the Marker picture to obtain the pose information O_{ref}'=(x, y, z, α, β, γ) of the reference point O_{ref} of the reference coordinate system in the current device coordinate system. Here, x, y, and z are the position information of the reference point O_{ref}', and α, β, and γ are the orientation information of the reference point O_{ref}'.

S203, establish a mapping relationship between the reference coordinate system and the device coordinate system according to the pose information of the reference point in the current device coordinate system.

Combined with the pose information of O_{ref}', a translation matrix T and a rotation matrix R from O_{dev} to O_{ref} are constructed by using coordinate basis transformation, and a transformation matrix M from the reference coordinate system Coord_{ref} to the device coordinate system Coord_{dev} is obtained by multiplying two matrices, that is, T·R.

The transformation matrix M is inverted to obtain M⁻¹. This matrix M⁻¹ is the transformation matrix from the device coordinate system Coord_{dev} to the reference coordinate system Coord_{ref}.

The transformation matrix M and M⁻¹ represent the mapping relationship between the reference coordinate system and the device coordinate system.

S204, measure positioning data of the physical object existing in the real space in the reference coordinate system.

The positioning data of the physical object existing in the real space, such as each wall, floor, ceiling, door, etc., relative to the reference point O_{ref}, is measured. The positioning data is used by the situation observation server to construct a virtual space Rᵥᵢᵣₜᵤₐₗ, so that each wall, floor, ceiling, door, etc., in the virtual space Rᵥᵢᵣₜᵤₐₗ coincides with the position of the each wall, floor, ceiling, door, etc., in the real space Rᵣₑₐₗ.

S205, scan a positioning picture with a significant feature at a position of a positioning benchmark point of the dental chair of the diagnosis-treatment table in the real space, to acquire pose information of the dental chair of the diagnosis-treatment table in the device coordinate system.

The diagnosis-treatment tables (C₁, C₂, ..., Cₙ) including dental chairs for simulation training are pre-arranged in the real empty room Rᵣₑₐₗ. Different Marker picture Mᵢ is placed at the origin of each diagnosis-treatment table Cᵢ. Here, Mᵢ includes a picture of the dental chair of the diagnosis-treatment table in the real space.

The virtual-real fusion head-mounted display scans Mᵢ to obtain the Marker identifier and the pose information of the diagnosis-treatment table Cᵢ in the device coordinate system Coord_{dev}.

S206, obtain positioning data of the dental chair of the diagnosis-treatment table in the reference coordinate system according to the pose information of the dental chair of the diagnosis-treatment table in the device coordinate system and the mapping relationship.

The virtual-real fusion head-mounted display uses the transformation matrix M⁻¹ to transform the pose information of the dental chair of the diagnosis-treatment table in the device coordinate system to the reference coordinate system Coord_{ref}, and saves the pose information in combination with the Marker identifier, to obtain the positioning data of the dental chair of the diagnosis-treatment table in the reference coordinate system.

The virtual-real fusion head-mounted display communicates the acquired positioning data of the physical object existing in the real space and the dental chair of the diagnosis-treatment table to the situation observation server based on the TCP/IP protocol. The situation observation server generates a virtual space according to the positioning data of the physical object in the reference coordinate system, and generates a virtual dental chair of a diagnosis-treatment table at a corresponding position in the virtual space according to the positioning data of the dental chair of the diagnosis-treatment table in the reference coordinate system. In addition, in response to a user's operation, a preset virtual facility is generated in the virtual space, and then coordinate-pose data of the virtual space, the virtual dental chair of the diagnosis-treatment table, and the virtual facility is acquired, and a scene file for performing oral diagnosis-treatment is generated according to the coordinate-pose data.

In the above embodiment of the present application, the virtual-real fusion head-mounted display scans the positioning picture with the significant feature at the specified position in the real space to determine the reference coordinate system and the reference point, and acquires the pose information of the reference point in the current device coordinate system, and then establishes the mapping relationship between the reference coordinate system and the device coordinate system according to the pose information of the reference point in the current device coordinate system. The virtual-real fusion head-mounted display measures the positioning data of the physical object existing in the real space in the reference coordinate system, and scans the positioning picture with the significant feature at the position of the positioning benchmark point of the dental chair of the diagnosis-treatment table in the real space, to acquire the pose information of the dental chair of the diagnosis-treatment table in the device coordinate system, and then obtains the positioning data of the dental chair of the diagnosis-treatment table in the reference coordinate system according to the pose information of the dental chair of the diagnosis-treatment table in the device coordinate system and the mapping relationship. In this embodiment, by establishing the mapping relationship between the reference coordinate system Coord_{ref} and the device coordinate system Coord_{dev}, when the virtual-reality fusion head-mounted display performs visual display, it is convenient for the virtual-real fusion head-mounted display to utilize the mapping relationship between these two coordinate systems to achieve virtual-real fusion of spatial coordinates.

Furthermore, on the basis of these above embodiments, the following embodiment illustrates the process of the simulation computer obtaining the matched simulated entity data.

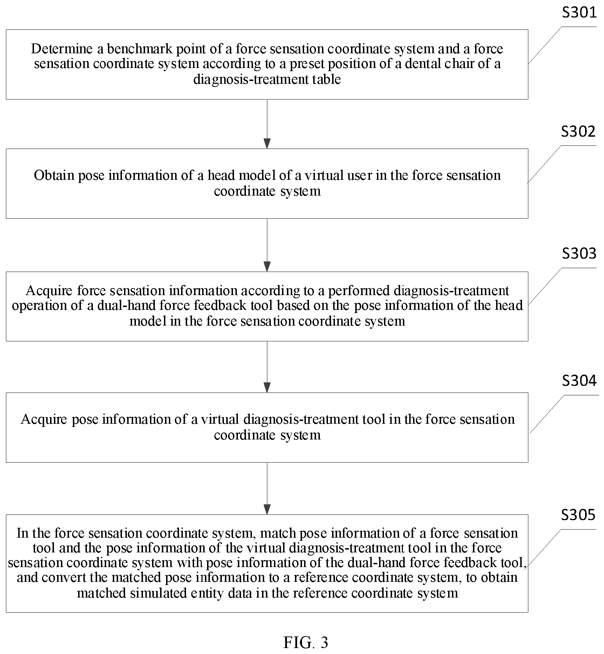

FIG. 3 is a schematic flow chart of a method for obtaining matched simulated entity data provided in an embodiment of the present application. As shown in FIG. 3, the method may include the following steps.

S301, determine a benchmark point of a force sensation coordinate system and the force sensation coordinate system according to a preset position of a dental chair of a diagnosis-treatment table.

A position is selected in advance within a real empty room Rᵣₑₐₗ as the reference point O_{ref}, and the orientation of each coordinate axis is specified to form the reference coordinate system Coord_{ref},

According to the method in the embodiment shown in FIG. 2, the mapping relationship between the reference coordinate system and the device coordinate system is established, that is, the transformation matrix M is constructed.

For any dental chair Cᵢ in the real space, a world transformation matrix M_{Ci} of the dental chair Cᵢ is acquired. The matrix includes the pose information of Ci in the reference coordinate system Coord_{ref}.

A midpoint of a connecting line (on a panel) of dual-hand force feedback inkwells fixed on a backrest of the dental chair Cᵢ is selected as a reference point of a haptic workspace, denoted as O_{Fi}. The pose of O_{Fi} in a local coordinate system of the dental chair is T_{L-Ofi}, and the pose of O_{Fi} in the reference coordinate system Coord_{ref} is T_{W-Ofi}=M_{Ci}·T_{L-Ofi}, denoted as M_{W-Ofi}. A force sensation coordinate system Coord_{Ofi} is established with O_{Fi} as the origin, and the inverse of M_{W-Ofi} is taken to obtain the transformation matrix M_{Ofi}⁻¹ from Coord_{ref} to Coord_{Ofi}.

S302, obtain pose information of a head model of a virtual patient in the force sensation coordinate system.

When constructing the virtual patient with force feedback interaction capability, the simulation computer records local pose information T_{L-HB} of the coordinate origin of the head model with haptic sensation relative to an animation bone Head Bone.

When the real user sends a control instruction through a microphone, and the simulation computer controls the virtual patient to lie on the dental chair Cᵢ according to the control instruction, the simulation computer acquires the pose information T_{W-HB} of the Head Bone in the reference coordinate system Coord_{ref}, calculates the pose information T_{H-Ref}=T_{W-HB}·T_{L-HB} of the force-sensation head model in Coord_{ref}, and transforms the pose information to the force sensation coordinate system Coord_{Ofi} to obtain the pose information T_{H-Ofi}=M_{Ofi}⁻¹·T_{H-Ref} in the force sensation coordinate system.

S303, acquire force sensation information according to a performed diagnosis-treatment operation of a dual-hand force feedback tool based on the pose information of the head model in the force sensation coordinate system.

The simulation computer transmits the pose information T_{H-Ofi} of the force-sensation head model to a force feedback module. The real user uses the dual-hand force feedback tool to perform the diagnosis-treatment operation, driving the force feedback module to perform force sensation calculation to obtain the force sensation information. The force sensation information includes the pose information of the force sensation tool and the magnitude and direction of the force, and the calculated force sensation information is fed back to the real user through the dual-hand force feedback tool.

S304, acquire pose information of a virtual diagnosis-treatment tool in the force sensation coordinate system.

Handles of the dual-hand force feedback tool (tools of the left and right force feedback devices) are respectively translated from the inkwell to O_{Fi} to obtain the pose information of the respective handles in the respective device coordinate systems, denoted as T_{L} and T_{R} respectively. The inverses of T_{L} and T_{R} are taken respectively to obtain the transformation matrices T_{L}⁻¹ and T_{R}⁻¹, that is, the respective transformation matrices from the respective force feedback device coordinate systems to the force sensation coordinate system Coord_{Ofi}.

S305, in the force sensation coordinate system, match the pose information of the force sensation tool and the pose information of the virtual diagnosis-treatment tool in the force sensation coordinate system with pose information of the dual-hand force feedback tool, and convert the matched pose information to the reference coordinate system, to obtain matched simulated entity data in the reference coordinate system; where the matched simulated entity data is used by the virtual-real fusion head-mounted display to generate a virtual-real fusion diagnosis-treatment visual with better matching effect.

The transformation matrices T_{L}⁻¹ and T_{R}⁻¹ are used to transform the respective dual-hand force feedback diagnosis-treatment tools to the force sensation coordinate system Coord_{Ofi}, to obtain the pose information P_{Ti-Ofi} in the force sensation coordinate system, where i may be L or R.

The pose information P_{Ti-Ofi} is transformed to the reference coordinate system Coord_{ref}, to obtain the pose information P_{Ti-Ref}=T_{W-Ofi}·P_{Ti-Ofi} in the reference coordinate system.

The P_{Ti-Ref} is transformed to the device coordinate system for the virtual-real fusion head-mounted display by using the transformation matrix M, to obtain the simulated entity data in the device coordinate system. The virtual-real fusion head-mounted display obtains the scene file, obtains the virtual visual in the virtual-real fusion space according to the scene file and the simulated entity data, and obtains a real-time visual captured in the real space, then performs superposition processing on the virtual visual in the virtual-real fusion space and the real-time visual, finally obtains the virtual-real fusion diagnosis-treatment visual, thus achieving triple matching of the force sensation of the diagnosis-treatment tool, the virtual diagnosis-treatment tool, and the dual-hand force feedback tool in the fusion space.

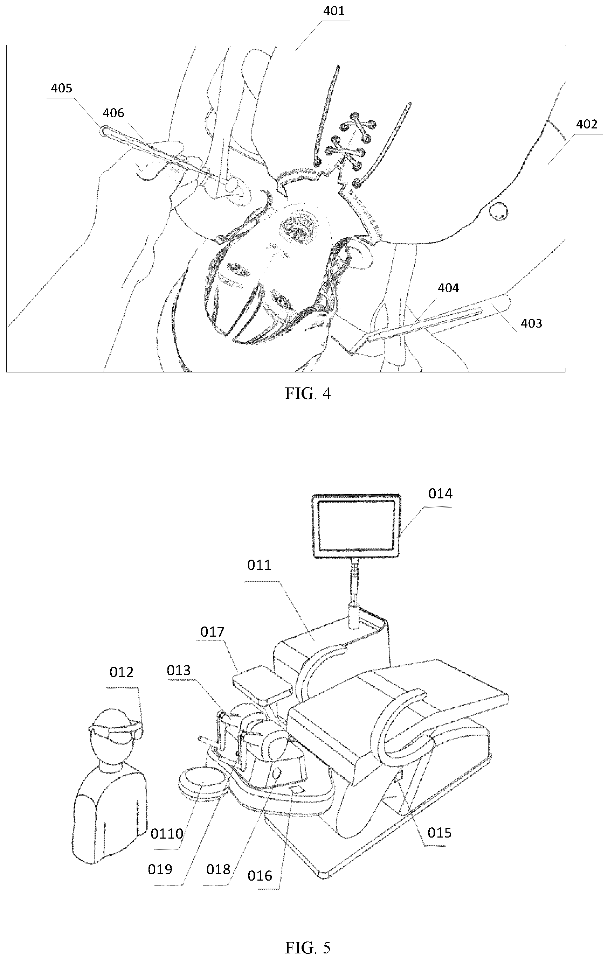

As shown in FIG. 4, FIG. 4 is a schematic diagram after virtual-real matching provided in an embodiment of the present application. In FIG. 4, 401 is a virtual patient, 402 is a physical dental chair, 403 is a handle of a right-hand force feedback device, 404 is a right-hand virtual diagnosis-treatment tool, 405 is a handle of a left-hand force feedback device, and 406 is a left-hand virtual diagnosis-treatment tool. It can be seen from FIG. 4 that the force feedback physical handle and the virtual diagnosis-treatment tool are coincidentally matched in a virtual-real fusion space. In terms of actual haptic sensation, the virtual diagnosis-treatment tool is also matched with the force sensation tool model, where a real user perceives force through the dual-hand force feedback device based on the force sensation tool model.

In the above embodiment of the present application, by determining a reference point of a force sensation coordinate system and the force sensation coordinate system according to a preset position of the dental chair of a diagnosis-treatment table, pose information of a head model of the virtual patient in the force sensation coordinate system is acquired, and a diagnosis-treatment operation is performed according to the pose information of the head model in the force sensation coordinate system through the dual-hand force feedback tool to acquire force sensation information. In addition, pose information of the virtual diagnosis-treatment tool in the force sensation coordinate system is acquired, and in the force sensation coordinate system, pose information of the force sensation tool and the pose information of the virtual diagnosis-treatment tool in the force sensation coordinate system are matched with pose information of the dual-hand force feedback tool, and the matched pose information is converted to a reference coordinate system, to obtain matched simulated entity data in the reference coordinate system. In this embodiment, by matching the force sensation tool and the virtual diagnosis-treatment tool with the dual-hand force feedback tool, the matching of a force sensation space, a virtual space, and a real space is realized, making a virtual-real fusion diagnosis-treatment visual generated in a virtual-real fusion head-mounted display more accurate.

Next, through the following embodiment, a more detailed structural composition of the dental simulation workbench training system of the present application based on spatial computing technology will be described.

In this embodiment, in addition to the simulation computer 011, the virtual-real fusion head-mounted display 012, and the dual-hand force feedback tool 013, the diagnosis-treatment table 01 further includes: a touch display 014, a dental chair height sensor 015, a pitch sensor 016, a dental chair height/pitch controller panel 017, a speaker 018, a microphone 019, and a retractable headrest assembly 0110.

As shown in FIG. 5, FIG. 5 is a schematic diagram of a dental simulation workbench 01 training system based on spatial computing technology provided in an embodiment of the present application.

Here, the dental chair of the diagnosis-treatment table 01 features a lifting function and a backrest pitching function. The side of the dental chair is designed as a frame that can house a host-machine of the diagnosis-treatment table 01. The mechanical structure of the dental chair can stably support the entire diagnosis-treatment table 01. Two connection driving rods are used to respectively control the lifting of the dental chair and the pitching of the backrest. Each driving rod is connected to a motor, and controlled by the lifting/pitching controller panel 017.

The dual-hand force feedback tool 013 is composed of two force feedback devices, which are fixed on the backrest of the dental chair, and are symmetrically arranged along the longitudinal central axis of the dental chair. Sufficient space is reserved to prevent physical interference between the devices, which can be lifted and pitched synchronously with the dental chair.

For the touch display 014, the display and the frame housing the simulation computer are connected through a 6-axis connecting piece, allowing a user to use the display at any height, left-right angle, and pitch angle.

The dental chair height sensor 015 and the pitch sensor 016 use inclination angle sensors to measure the real-time angle between the supporting rod of the dental chair and a horizontal plane. The height sensor 015 is fixed on the supporting rod of the main frame, and the pitch sensor 016 is fixed on the backrest.

The height/pitch controller panel of the dental chair is connected with the host-machine of the dental chair through a 2-axis connecting piece, which can provide left-right overall deflection of the controller panel 017 and a connecting rod, and rotation of the panel itself, so as to meet user requirements.

For the speaker 018 and the microphone 019, mature industrial devices for speakers and microphones are selected. Corresponding installation positions are designed, so that the speaker 018 is installed inside the backrest of the dental chair, and the microphone 019 is installed in the middle of the dual-hand force feedback. A hole is made on the panel, so that the microphone 019 can receive sound.

The retractable headrest assembly 0110 may be adjusted according to virtual patients of different heights, so that the head of a virtual patient is just located at a suitable position of the headrest, providing support during real user operations.

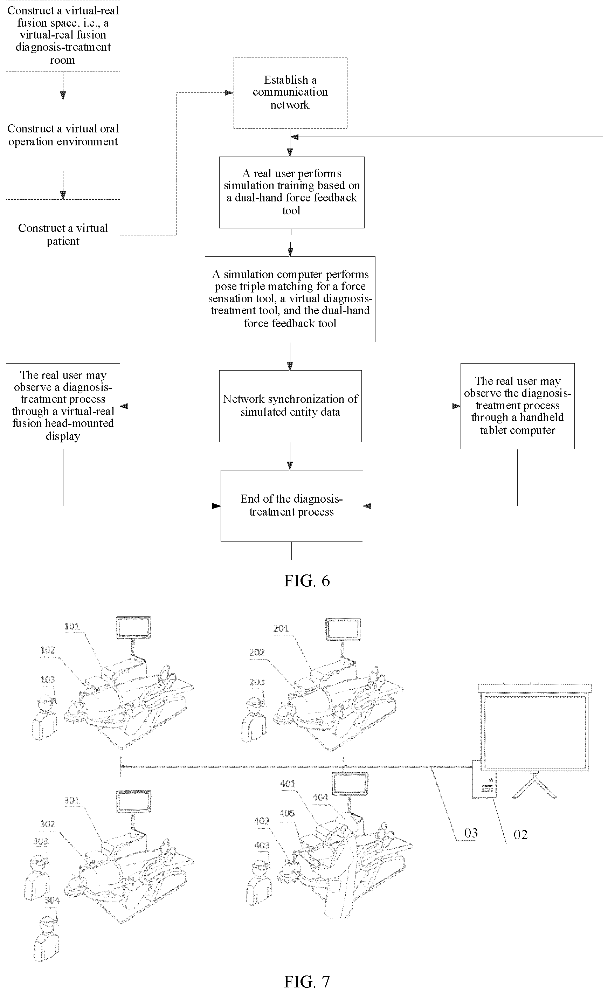

For the convenience of understanding the above dental simulation workbench training system based on spatial computing technology of the present application, the following takes a specific example to illustrate its application process. FIG. 6 is a schematic application diagram of a dental simulation workbench training system based on spatial computing technology provided in an embodiment of the present application. As shown in FIG. 6, the application process includes a preparation stage and a training stage.

In the preparation stage (as shown by the dotted line in FIG. 6), the following occurs.

Construct a virtual-real fusion space, i.e., a virtual-real fusion diagnosis-treatment room:
the constructed virtual-real fusion diagnosis-treatment room includes a virtual object corresponding to a physical object existing in a real space, a virtual dental chair of a diagnosis-treatment table, and virtual objects such as a virtual medical cabinet and a virtual number-calling screen.

Construct a virtual oral operation environment:
a virtual oral model is constructed from oral data such as CBCT scan data and true color scan data, a virtual diagnosis-treatment tool is constructed from measurement data of a diagnosis-treatment tool, and the virtual oral operation environment is constructed from the virtual oral model and the virtual diagnosis-treatment tool.

Construct a virtual patient:
a digital holographic real-time three-dimensional model with voice recognition interaction is used to define an instruction-based behavior-driven model, a diagnosis-treatment information set for the virtual patient is constructed from the behavior-driven model and a knowledge graph model, and the diagnosis-treatment information set is fused with the virtual oral operation environment to generate the virtual patient. The virtual patient is able to receive a control instruction of a simulation computer to complete behaviors such as walking, standing, getting on/off the dental chair, and facial expression control.

Establish a communication network:
communication between a plurality of diagnosis-treatment tables, that is, a plurality of simulation computers, and a situation observation server, as well as communication between a virtual-real fusion head-mounted display and the situation observation server, are implemented. Here, the simulation computers may be connected to the situation observation server in a wired manner, and the virtual-real fusion head-mounted display may be connected to the situation observation server in a wireless manner, with respective TCP/IP communication links established.

In the simulation training stage (as shown by the solid line in FIG. 6), the following occurs.

A real user performs simulation training based on a dual-hand force feedback tool:
the simulation computer loads a scene file distributed by the situation observation server to generate the virtual-real fusion space. The real user performs a number-calling operation through an operation interface UI of a touch display to generate the virtual patient, performs an interrogation with the generated virtual patient through a microphone in a voice manner, and receives an interactive voice output by the virtual patient through a speaker.

The real user uses the dual-hand force feedback tool as an operation medium to perform diagnosis-treatment operations in the virtual-real fusion space. The simulation computer collects position data of the dual-hand force feedback tool in real time to calculate force sensation information, and feeds the calculated force sensation information back to the operator, that is, the real user, through the dual-hand force feedback tool, so that the operator feels the force sensation of the virtual scene. Here, the force sensation information includes pose information of a force sensation tool, and magnitude and direction of a force.

The simulation computer performs pose triple matching for the force sensation tool, the virtual diagnosis-treatment tool, and the dual-hand force feedback tool:
the simulation computer performs pose matching for the force sensation tool, the virtual diagnosis-treatment tool, and the dual-hand force feedback tool based on a preset matching algorithm to ensure that the poses of the virtual diagnosis-treatment tool in terms of force sensation and vision always coincide with those of the dual-hand force feedback tool.

After the lifting and pitching states of the dental chair are adjusted, the virtual patient follows the dental chair to lift and pitch synchronously. When the simulation computer acquires that the lifting height of the dental chair measured by a height sensor or the rotation angle of the dental chair measured by a pitch sensor changes, based on the above matching algorithm, the simulation computer controls the pose of the force sensation tool and the pose of the virtual diagnosis-treatment tool to match with the pose of the real diagnosis-treatment tool again. At this time, new pose T_{L-Ofi}' of O_{Fi} in a local coordinate system of the dental chair is re-acquired, and M_{W-Ofi} and M_{Ofi}⁻¹ are updated, the pose information of the virtual diagnosis-treatment tool in the virtual-real fusion head-mounted display is updated, then the triple matching is implemented again to obtain new simulated entity data.

Network synchronization of the simulated entity data:
after a simulation server acquires the matched simulated entity data from the simulation computer, the simulation server may process or forward the simulated entity data according to a corresponding strategy. Each piece of simulated entity data is provided with a global unique identifier Id_{Global} and other associated information, such as entity type, entity pose, simulation grid deformation data, and animation data, etc.

The real user may observe a diagnosis-treatment process through the virtual-real fusion head-mounted display:
the virtual-real fusion head-mounted display scans a Marker picture to determine a reference coordinate system and a reference point, and establishes a mapping relationship between the reference coordinate system and a device coordinate system, that is, the mapping relationship between a virtual space and a real space. The scene file is loaded, the matched simulated entity data sent by the simulation computer is received, a virtual visual in the virtual-real fusion space is obtained, and finally, after a real-time visual of the real space is superimposed, a virtual-real fusion diagnosis-treatment visual is obtained.

The real user may observe the diagnosis-treatment process through a handheld tablet computer:
in the present application, the dental simulation workbench system based on spatial computing technology may further be connected to at least one external handheld terminal, so that the external handheld terminal displays the virtual-real fusion diagnosis-treatment visual. Here, the external handheld terminal has an augmented reality function and is connected to the simulation computer in a wireless manner. Taking a handheld tablet computer as an example of the external handheld terminal, the handheld tablet computer scans the Marker picture to determine the reference coordinate system and the reference point, and establishes the mapping relationship between the reference coordinate system and the device coordinate system, that is, the mapping relationship between the virtual space and the real space. The scene file is loaded, the matched simulated entity data sent by the simulation computer is received, the virtual visual in the virtual-real fusion space is obtained, and finally, after the real-time visual of the real space is superimposed, the virtual-real fusion diagnosis-treatment visual is obtained.

The number of connectable external handheld terminals in the present application is not limited, which may be one or more.

End of the diagnosis-treatment process:
after the diagnosis-treatment process ends, the simulation computer may further control the virtual patient to leave the diagnosis-treatment table, and provide a systematic evaluation of the diagnosis-treatment operation of the real user in the virtual-real fusion space.

In the present application, a plurality of diagnosis-treatment tables are connected to a situation observation server. As shown in FIG. 7, FIG. 7 is a schematic scene diagram provided in an embodiment of the present application. In FIG. 7, there is the following.

It is assumed that there are 4 diagnosis-treatment tables in a real diagnosis-treatment space, and the four diagnosis-treatment tables are connected to a situation observation server 02 through a local area wired network 03, where the situation observation server 02 is equipped with a large display screen. 101 is the No. 1 diagnosis-treatment table, 102 is the virtual patient of the No. 1 diagnosis-treatment table, and 103 is the real user of the No. 1 diagnosis-treatment table. Similarly, 201 is the No. 2 diagnosis-treatment table, 202 is the virtual patient of the No. 2 diagnosis-treatment table, and 203 is the real user of the No. 2 diagnosis-treatment table. 301 is the No. 3 diagnosis-treatment table, 302 is the virtual patient of the No. 3 diagnosis-treatment table, 303 is the real user of the No. 3 diagnosis-treatment table, and 304 is another real user of this diagnosis-treatment table. In the No. 4 diagnosis-treatment table, in addition to 401, 402, and 403 being the No. 4 diagnosis-treatment table, the virtual patient, and the real user, respectively, there is further 404, which is a teacher, who may observe the diagnosis-treatment process of the No. 4 diagnosis-treatment table through a handheld tablet computer 405.

In the present application, through the situation observation server, the real user may observe the diagnosis-treatment process of any diagnosis-treatment table. By controlling a "camera" function on the large display screen of the situation observation server, a "camera" is controlled to move to a preset position of a corresponding diagnosis-treatment table for viewing.

In the dental simulation workbench training system based on spatial computing technology of the present application, the simulation fidelity is improved by generating a virtual-real fusion diagnosis-treatment scene. Moreover, the system can support multiple people to watch the diagnosis-treatment process of the same diagnosis-treatment table, facilitating users to communicate and enhancing the user experience.

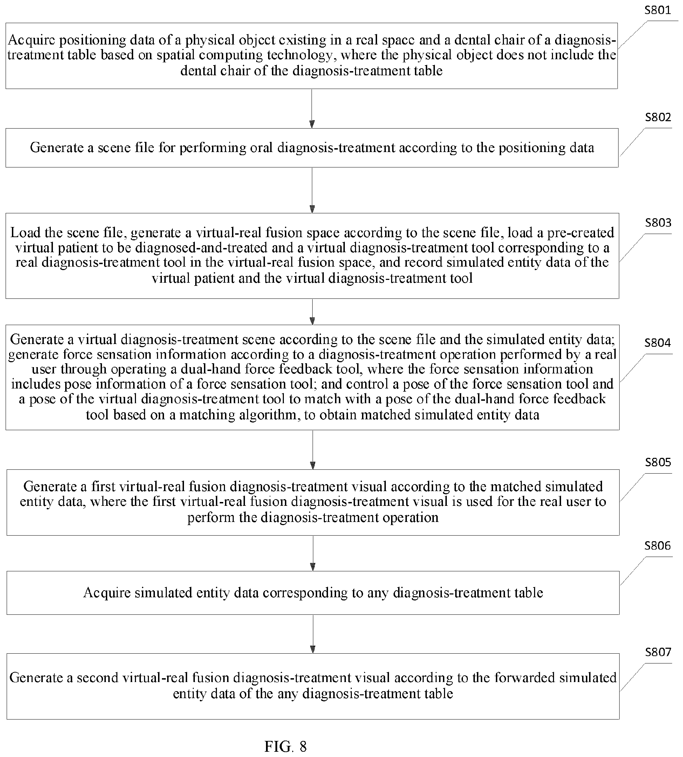

The present application further provides an oral diagnosis-treatment training method based on spatial computing technology, applied to a dental simulation workbench training system based on spatial computing technology. As shown in FIG. 8, FIG. 8 is a schematic flow chart of an oral diagnosis-treatment training method based on spatial computing technology provided in an embodiment of the present application. The method includes the following.

S801, acquire positioning data of a physical object existing in a real space and a dental chair of a diagnosis-treatment table based on spatial computing technology, where the physical object does not include the dental chair of the diagnosis-treatment table.

A possible implementation is:
a positioning picture with a significant feature at a specified position in the real space is scanned to determine a reference coordinate system and a reference point, and pose information of the reference point is acquired in a current device coordinate system. A mapping relationship between the reference coordinate system and the device coordinate system is established according to the pose information of the reference point in the current device coordinate system. In addition, positioning data of the physical object existing in the real space in the reference coordinate system is measured, and a positioning picture with a significant feature at a position of a positioning benchmark point of the dental chair of the diagnosis-treatment table in the real space is scanned, to acquire pose information of the dental chair of the diagnosis-treatment table in the device coordinate system, thus positioning data of the dental chair of the diagnosis-treatment table in the reference coordinate system is obtained according to the pose information of the dental chair of the diagnosis-treatment table in the device coordinate system and the mapping relationship.

S802, generate a scene file for performing oral diagnosis-treatment according to the positioning data.

A possible implementation is:
a virtual space is generated according to the positioning data of the physical object in the reference coordinate system, a virtual dental chair of the diagnosis-treatment table is generated at a corresponding position in the virtual space according to the positioning data of the dental chair of the diagnosis-treatment table in the reference coordinate system, and a preset virtual facility is generated in the virtual space in response to a user's operation. Coordinate-pose data of the virtual space, the virtual dental chair of the diagnosis-treatment table, and the virtual facility is acquired, and the scene file for performing oral diagnosis-treatment is generated according to the coordinate-pose data.

S803, load the scene file, generate a virtual-real fusion space according to the scene file, load a pre-created virtual patient to be diagnosed-and-treated and a virtual diagnosis-treatment tool corresponding to a real diagnosis-treatment tool in the virtual-real fusion space, and record simulated entity data of the virtual patient and the virtual diagnosis-treatment tool.

S804, generate a virtual diagnosis-treatment scene according to the scene file and the simulated entity data; generate force sensation information according to a diagnosis-treatment operation performed by a real user through operating a dual-hand force feedback tool, where the force sensation information includes pose information of a force sensation tool; and control a pose of the force sensation tool and a pose of the virtual diagnosis-treatment tool to match with a pose of the dual-hand force feedback tool based on a matching algorithm, to obtain matched simulated entity data.

A possible implementation is:
a benchmark point of a force sensation coordinate system and the force sensation coordinate system are determined according to a preset position of the dental chair of the diagnosis-treatment table, and pose information of a head model of the virtual patient in the force sensation coordinate system is acquired. The force sensation information is generated according to the performed diagnosis-treatment operation of the dual-hand force feedback tool based on the pose information of the head model in the force sensation coordinate system, where the force sensation information includes the pose information of the force sensation tool. The pose information of the virtual diagnosis-treatment tool in the force sensation coordinate system is acquired, and in the force sensation coordinate system, the pose information of the force sensation tool and the pose information of the virtual diagnosis-treatment tool in the force sensation coordinate system are matched with the pose information of the dual-hand force feedback tool, and the matched pose information is converted to the reference coordinate system, to obtain the matched simulated entity data in the reference coordinate system.

S805, generate a first virtual-real fusion diagnosis-treatment visual according to the matched simulated entity data, where the first virtual-real fusion diagnosis-treatment visual is used for the real user to perform the diagnosis-treatment operation.

A possible implementation is:
the mapping relationship between the reference coordinate system and the device coordinate system is acquired, and the matched simulated entity data in the reference coordinate system is converted to the device coordinate system according to the mapping relationship, to obtain simulated entity data in the device coordinate system. The scene file is acquired, a virtual visual in the virtual-real fusion space is obtained according to the scene file and the simulated entity data in the device coordinate system, and a real-time visual captured in the real space is acquired. Superposition processing is performed on the virtual visual in the virtual-real fusion space and the real-time visual, and finally the first virtual-real fusion diagnosis-treatment visual is obtained.

S806, acquire simulated entity data corresponding to any diagnosis-treatment table.

S807, generate a second virtual-real fusion diagnosis-treatment visual according to the forwarded simulated entity data of the any diagnosis-treatment table.

Here, in both the first virtual-real fusion diagnosis-treatment visual and the second virtual-real fusion diagnosis-treatment visual, the virtual patient undergoing the diagnosis-treatment operation is shown to be closely aligned with the dental chair of the diagnosis-treatment table in the real space.

In the above method of the present application, for the specific implementation process and technical effects, please refer to the above embodiments. To avoid redundancy, no further description will be given.

The present application further provides a computer program product including a computer program, and when the computer program is executed by a processor, the above method is implemented.

The present application further provides a computer-readable storage medium, where computer execution instructions are stored in the computer-readable storage medium, and when a processor executes the computer execution instructions, the above method is implemented.

A unit described as a separate component may or may not be physically separate, and a component displayed as a unit may or may not be a physical unit, that is, it may be located in one place, or may be distributed on a plurality of network units. Some or all of the units may be selected according to actual needs to achieve the purpose of the solution of this embodiment.

In addition, functional units in the embodiments of the present application may be integrated into one processing unit, or each unit may exist alone physically, or two or more units may be integrated into one unit.

If a function is implemented in the form of a software functional unit and sold or used as an independent product, it may be stored in a computer-readable storage medium. Based on this understanding, the technical solution of the present application, in essence, or a part contributing to the prior art, or a part of this technical solution, may be embodied in the form of a software product. This computer software product is stored in a storage medium, and includes several instructions for enabling a computer device (which may be a personal computer, a server, or a network device, etc.) to execute all or part of the steps of the methods described in the embodiments of the present application. The foregoing storage medium includes: any medium that can store program codes, such as a USB flash drive, a removable hard disk, a Read-Only Memory (ROM, Read-Only Memory), a Random Access Memory (RAM, Random Access Memory), a magnetic disk, or an optical disc.

A person of ordinary skill in the art can understand that all or part of the steps for implementing the foregoing method embodiments may be completed by hardware related to program instructions. The foregoing program may be stored in a computer-readable storage medium. When the program is executed, the steps including the foregoing method embodiments are executed; and the foregoing storage medium includes: various media that can store program codes, such as a ROM, a RAM, a magnetic disk, or an optical disc.

Finally, it should be noted that: after considering the description and practicing the invention disclosed herein, those skilled in the art will easily think of other implementations of the present application. The present application is intended to cover any variations, uses, or adaptations of the present application, which follow the general principles of the present application and include common knowledge or conventional technical means in the technical field not disclosed in the present application. The present application is not limited to the precise structure already described above and shown in the drawings, and various modifications and changes can be made without departing from the scope thereof. The scope of the present application is only limited by the appended claims.

## Claims

1. A dental simulation workbench training system based on spatial computing technology, comprising: a plurality of diagnosis-treatment tables and a situation observation server; wherein the plurality of diagnosis-treatment tables communicate with the situation observation server through a TCP/IP protocol, and each of the diagnosis-treatment tables comprises: a simulation computer, a virtual-real fusion head-mounted display, and a dual-hand force feedback tool;
the virtual-real fusion head-mounted display is configured to acquire positioning data of a physical object existing in a real space and a dental chair of a diagnosis-treatment table based on spatial computing technology, wherein the physical object does not comprise the dental chair of the diagnosis-treatment table;
the situation observation server is configured to generate a scene file for performing oral diagnosis-treatment according to the positioning data;
the simulation computer is configured to: load the scene file, generate a virtual-real fusion space according to the scene file, load a pre-created virtual patient to be diagnosed-and-treated and a virtual diagnosis-treatment tool corresponding to a real diagnosis-treatment tool in the virtual-real fusion space, and record simulated entity data of the virtual patient and the virtual diagnosis-treatment tool;
the simulation computer is further configured to: generate a virtual diagnosis-treatment scene according to the scene file and the simulated entity data; generate force sensation information according to a diagnosis-treatment operation performed by a real user through operating the dual-hand force feedback tool, wherein the force sensation information comprises pose information of a force sensation tool; and control a pose of the force sensation tool and a pose of the virtual diagnosis-treatment tool to match with a pose of the dual-hand force feedback tool based on a matching algorithm, to obtain matched simulated entity data;
the virtual-real fusion head-mounted display is configured to generate a first virtual-real fusion diagnosis-treatment visual according to the matched simulated entity data, wherein the first virtual-real fusion diagnosis-treatment visual is used for the real user to perform the diagnosis-treatment operation;
the situation observation server is further configured to acquire simulated entity data corresponding to any one of the diagnosis-treatment tables;
the virtual-real fusion head-mounted display is further configured to generate a second virtual-real fusion diagnosis-treatment visual according to the simulated entity data of the any one of the diagnosis-treatment tables forwarded by the situation observation server;
wherein in both the first virtual-real fusion diagnosis-treatment visual and the second virtual-real fusion diagnosis-treatment visual, the virtual patient undergoing the diagnosis-treatment operation is shown to be closely aligned with the dental chair of the diagnosis-treatment table in the real space.

2. The system according to claim 1, wherein when configured to acquire the positioning data of the physical object existing in the real space and the dental chair of the diagnosis-treatment table based on spatial computing technology, the virtual-real fusion head-mounted display is specifically configured to:
scan a positioning picture with a significant feature at a specified position in the real space to determine a reference coordinate system and a reference point;
acquire pose information of the reference point in a current device coordinate system;
establish a mapping relationship between the reference coordinate system and the device coordinate system according to the pose information of the reference point in the current device coordinate system;
measure positioning data of the physical object existing in the real space in the reference coordinate system;
scan a positioning picture with a significant feature at a position of a positioning benchmark point of the dental chair of the diagnosis-treatment table in the real space, to acquire pose information of the dental chair of the diagnosis-treatment table in the device coordinate system;
obtain positioning data of the dental chair of the diagnosis-treatment table in the reference coordinate system according to the pose information of the dental chair of the diagnosis-treatment table in the device coordinate system and the mapping relationship.

3. The system according to claim 2, wherein when configured to generate the scene file for performing oral diagnosis-treatment according to the positioning data, the situation observation server is specifically configured to:
generate a virtual space according to the positioning data of the physical object in the reference coordinate system;
generate a virtual dental chair of the diagnosis-treatment table at a corresponding position in the virtual space according to the positioning data of the dental chair of the diagnosis-treatment table in the reference coordinate system;
generate a preset virtual facility in the virtual space in response to a user's operation;
acquire coordinate-pose data of the virtual space, the virtual dental chair of the diagnosis-treatment table, and the virtual facility, and generate the scene file for performing oral diagnosis-treatment according to the coordinate-pose data.

4. The system according to claim 3, wherein when configured to generate the force sensation information according to the diagnosis-treatment operation performed by the real user through operating the dual-hand force feedback tool, wherein the force sensation information comprises the pose information of the force sensation tool, and control the pose of the force sensation tool and the pose of the virtual diagnosis-treatment tool to match with the pose of the dual-hand force feedback tool based on the matching algorithm, to obtain the matched simulated entity data, the simulation computer is specifically configured to:
determine a benchmark point of a force sensation coordinate system and the force sensation coordinate system according to a preset position of the dental chair of the diagnosis-treatment table;
acquire pose information of a head model of the virtual patient in the force sensation coordinate system;
generate the force sensation information according to the performed diagnosis-treatment operation of the dual-hand force feedback tool based on the pose information of the head model in the force sensation coordinate system, wherein the force sensation information comprises the pose information of the force sensation tool;
acquire pose information of the virtual diagnosis-treatment tool in the force sensation coordinate system;
in the force sensation coordinate system, match the pose information of the force sensation tool and the pose information of the virtual diagnosis-treatment tool in the force sensation coordinate system with pose information of the dual-hand force feedback tool, and convert the matched pose information to the reference coordinate system, to obtain matched simulated entity data in the reference coordinate system.

5. The system according to claim 4, wherein when configured to generate the first virtual-real fusion diagnosis-treatment visual according to the matched simulated entity data, the virtual-real fusion head-mounted display is specifically configured to:
acquire the mapping relationship between the reference coordinate system and the device coordinate system;
convert the matched simulated entity data in the reference coordinate system to the device coordinate system according to the mapping relationship, to obtain simulated entity data in the device coordinate system;
acquire the scene file, and obtain a virtual visual in the virtual-real fusion space according to the scene file and the simulated entity data in the device coordinate system;
acquire a real-time visual captured in the real space;
perform superposition processing on the virtual visual in the virtual-real fusion space and the real-time visual, to obtain the first virtual-real fusion diagnosis-treatment visual.

6. The system according to claim 1, wherein the situation observation server is further configured to:
display virtual diagnosis-treatment scenes corresponding to different diagnosis-treatment tables.

7. The system according to claim 1, wherein the situation observation server is further configured to:
set a virtual patient number-calling mode, wherein the number-calling mode comprises a normal number-calling mode and an enhanced number-calling mode, wherein a virtual patient corresponding to the normal number-calling mode has a random disease cause, and a virtual patient corresponding to the enhanced number-calling mode has a preset fixed disease cause;
edit a virtual patient queue corresponding to the number-calling mode.

8. The system according to claim 1, wherein the diagnosis-treatment table further comprises: a speaker and a microphone;
after the simulation computer loads the pre-created virtual patient to be diagnosed-and-treated in the virtual-real fusion space, the real user sends a control instruction to the virtual patient through the microphone, and receives an interactive voice output by the virtual patient through the speaker.

9. The system according to claim 1, wherein the diagnosis-treatment table further comprises: a height sensor and a pitch sensor;
when the simulation computer detects that a lifting height of the dental chair measured by the height sensor or a rotation angle of the dental chair measured by the pitch sensor changes, the simulation computer controls, based on the matching algorithm, the pose of the force sensation tool and the pose of the virtual diagnosis-treatment tool to match with the pose of the dual-hand force feedback tool again, to obtain new matched simulated entity data.

10. The system according to any one of claims 1-9, wherein the dental simulation workbench training system based on spatial computing technology is connected to at least one external handheld terminal, so that the at least one external handheld terminal displays a virtual-real fusion diagnosis-treatment visual, wherein the at least one external terminal has an augmented reality function.

11. An oral diagnosis-treatment training method based on spatial computing technology, applied to an oral diagnosis-treatment training system based on spatial computing technology, wherein the method comprises:
acquiring positioning data of a physical object existing in a real space and a dental chair of a diagnosis-treatment table based on spatial computing technology, wherein the physical object does not comprise the dental chair of the diagnosis-treatment table;
generating a scene file for performing oral diagnosis-treatment according to the positioning data;
loading the scene file, generating a virtual-real fusion space according to the scene file, loading a pre-created virtual patient to be diagnosed-and-treated and a virtual diagnosis-treatment tool corresponding to a real diagnosis-treatment tool in the virtual-real fusion space, and recording simulated entity data of the virtual patient and the virtual diagnosis-treatment tool;
generating a virtual diagnosis-treatment scene according to the scene file and the simulated entity data; generating force sensation information according to a diagnosis-treatment operation performed by a real user through operating a dual-hand force feedback tool, wherein the force sensation information comprises pose information of a force sensation tool; and controlling a pose of the force sensation tool and a pose of the virtual diagnosis-treatment tool to match with a pose of the dual-hand force feedback tool based on a matching algorithm, to obtain matched simulated entity data;
generating a first virtual-real fusion diagnosis-treatment visual according to the matched simulated entity data, wherein the first virtual-real fusion diagnosis-treatment visual is used for the real user to perform the diagnosis-treatment operation;
acquiring simulated entity data corresponding to any diagnosis-treatment table;
generating a second virtual-real fusion diagnosis-treatment visual according to the forwarded simulated entity data of the any diagnosis-treatment table;
wherein in both the first virtual-real fusion diagnosis-treatment visual and the second virtual-real fusion diagnosis-treatment visual, the virtual patient undergoing the diagnosis-treatment operation is shown to be closely aligned with the dental chair of the diagnosis-treatment table in the real space.

12. The method according to claim 11, wherein the acquiring the positioning data of the physical object existing in the real space and the dental chair of the diagnosis-treatment table based on spatial computing technology comprises:
scanning a positioning picture with a significant feature at a specified position in the real space to determine a reference coordinate system and a reference point;
acquiring pose information of the reference point in a current device coordinate system;
establishing a mapping relationship between the reference coordinate system and the device coordinate system according to the pose information of the reference point in the current device coordinate system;
measuring positioning data of the physical object existing in the real space in the reference coordinate system;
scanning a positioning picture with a significant feature at a position of a positioning benchmark point of the dental chair of the diagnosis-treatment table in the real space, to acquire pose information of the dental chair of the diagnosis-treatment table in the device coordinate system;
obtaining positioning data of the dental chair of the diagnosis-treatment table in the reference coordinate system according to the pose information of the dental chair of the diagnosis-treatment table in the device coordinate system and the mapping relationship.

13. The method according to claim 12, wherein the generating the scene file for performing oral diagnosis-treatment according to the positioning data comprises:
generating a virtual space according to the positioning data of the physical object in the reference coordinate system;
generating a virtual dental chair of the diagnosis-treatment table at a corresponding position in the virtual space according to the positioning data of the dental chair of the diagnosis-treatment table in the reference coordinate system;
generating a preset virtual facility in the virtual space in response to a user's operation;
acquiring coordinate-pose data of the virtual space, the virtual dental chair of the diagnosis-treatment table, and the virtual facility, and generating the scene file for performing oral diagnosis-treatment according to the coordinate-pose data.

14. The method according to claim 13, wherein the generating the force sensation information according to the diagnosis-treatment operation performed by the real user through operating the dual-hand force feedback tool, wherein the force sensation information comprises the pose information of the force sensation tool, and controlling the pose of the force sensation tool and the pose of the virtual diagnosis-treatment tool to match with the pose of the dual-hand force feedback tool based on the matching algorithm, to obtain the matched simulated entity data, comprises:
determining a benchmark point of a force sensation coordinate system and the force sensation coordinate system according to a preset position of the dental chair of the diagnosis-treatment table;
acquiring pose information of a head model of the virtual patient in the force sensation coordinate system;
generating the force sensation information according to the performed diagnosis-treatment operation of the dual-hand force feedback tool based on the pose information of the head model in the force sensation coordinate system, wherein the force sensation information comprises the pose information of the force sensation tool;
acquiring pose information of the virtual diagnosis-treatment tool in the force sensation coordinate system;
in the force sensation coordinate system, matching the pose information of the force sensation tool and the pose information of the virtual diagnosis-treatment tool in the force sensation coordinate system with pose information of the dual-hand force feedback tool, and converting the matched pose information to the reference coordinate system, to obtain matched simulated entity data in the reference coordinate system.

15. The method according to claim 14, wherein the generating the first virtual-real fusion diagnosis-treatment visual according to the matched simulated entity data comprises:
acquiring the mapping relationship between the reference coordinate system and the device coordinate system;
converting the matched simulated entity data in the reference coordinate system to the device coordinate system according to the mapping relationship, to obtain simulated entity data in the device coordinate system;
acquiring the scene file, and obtaining a virtual visual in the virtual-real fusion space according to the scene file and the simulated entity data in the device coordinate system;
acquiring a real-time visual captured in the real space;
performing superposition processing on the virtual visual in the virtual-real fusion space and the real-time visual, to obtain the first virtual-real fusion diagnosis-treatment visual.
